Europäisches Patentamt

European Patent Office

Office européen des brevets

(19)

(11) Veröffentlichungsnummer: **0 190 765**
**B1**

(12) **EUROPÄISCHE PATENTSCHRIFT**

(45) Veröffentlichungstag der Patentschrift: **04.07.90**

(21) Anmeldenummer: **86101607.9**

(22) Anmeldetag: **07.02.86**

(51) Int. Cl.⁵: **G 01 N 33/78**

(54) Verfahren zur Bestimmung der Bindungskapazität des Thyroxin bindenden Globulins.

(30) Priorität: **08.02.85 DE 3504406**

(43) Veröffentlichungstag der Anmeldung:
**13.08.86 Patentblatt 86/33**

(45) Bekanntmachung des Hinweises auf die
Patenterteilung:
**04.07.90 Patentblatt 90/27**

(84) Benannte Vertragsstaaten:
**AT BE CH DE FR GB IT LI LU NL SE**

(56) Entgegenhaltungen:
**GB-A-2 085 160**
**US-A-4 040 907**
**US-A-4 481 298**

**CLINICAL CHEMISTRY, Band 24, Nr. 6, 1978,
Seite 1033, Ref. 222; G. KLEINHAMMER et al.:
"Enzyme immuno assay for determination of the
thyroxin binding index"**

**Enzyme Labelled Immunoassay of Hormones
and Drugs, Walter de Gruyter, Berlin 1978, pp
153-174**

(73) Patentinhaber: **BOEHRINGER MANNHEIM
GMBH
Patentabteilung, Abt. E Sandhofer Strasse 112-
132 Postfach 31 01 20
D-6800 Mannheim 31 Waldhof (DE)**

(72) Erfinder: **Albert, Winfried, Dr. phil.
Moosstrasse 10
D-8121 Pähl (DE)**
Erfinder: **Draeger, Brigitte, Dr.rer.nat.
Am Bareisl 25
D-8132 Tutzing (DE)**
Erfinder: **Junius, Martina, Dr.rer.nat.
Eichenstrasse 4
D-8139 Bernried (DE)**

(74) Vertreter: **Weickmann, Heinrich, Dipl.-Ing. et al
Patentanwälte Dipl.-Ing. H.Weickmann Dipl.-
Phys.Dr. K.Fincke Dipl.-Ing. F.A.Weickmann
Dipl.-Chem. B. Huber Dr.-Ing. H. Liska Dipl.-
Phys.Dr. J. Prechtel Postfach 860820
D-8000 München 86 (DE)**

# EP 0 190 765 B1

**Beschreibung**

Die Erfindung betrifft ein heterogenes Verfahren zur Bestimmung der Bindungskapazität des Thyroxin bindenden Globulins (TBG), auch als T-Uptake bezeichnet.

Die Thyroid-Hormone liegen im Blut weitgehend in proteingebundener Form vor. Dabei bindet Thyroxin ($T_4$) insbesondere an das Thyroxin bindende Globulin (TBG). Die Bestimmung der Bindungskapazität von TBG ist im Rahmen der Schilddrüsendiagnostik von besonderer Bedeutung. Außerdem korreliert in der überwiegenden Anzahl der Fälle das Produkt aus Gesamt-$T_4$-Konzentration und 1/TBG gut mit der $FT_4$-Konzentration. (L. R. Witherspoon, Journal of Clinical Immunoassay 7, (1984)).

Bekannte Verfahren zur Bestimmung der TBG-Bindungskapazität sind in DE—A—28 25 650 der gleichen Anmelderin beschrieben. Der Nachteil der bekannten Verfahren liegt darin, radioaktive Markierungen verwenden zu müssen und/oder lange Inkubationszeiten (z.B. 2 Stunden) in Kauf nehmen zu müssen. Das Verfahren der DE—A—28 25 560 weist ebenfalls noch gewisse Nachteile auf die darauf beruhen, daß es sich um ein kompetitives Verfahren handelt und sowohl $T_4$ als auch $T_4$-Enzym-Konjugat zugegeben werden müssen.

Das Verfahren, das aus Clin Chem (1978) *24*(6) 1033 Ref. 222 und Enzyme Labelled Immunoassay of Hormones and Drugs, Walter de Gruyter, Berlin 1978, 153—174 bekannt ist, beruht auf dem Prinzip, daß man eine bekannte Menge $T_4$ zugibt, wobei ein Teil dieses zugegebenen $T_4$ die Bindungsstellen auf dem TBG besetzt. Das restliche freie $T_4$ konkurriert mit dem $T_4$-Konjugat um Bindung an den Anti-$T_4$-Antikörper und aus der Menge an gebundenem $T_4$-Konjugat kann dann auf die Menge an freiem $T_4$ bzw. die Menge an von TBG gebundenem $T_4$ und damit auf den TBI zurückgeschlossen werden. Dieses Verfahren hat vor allen Dingen den Nachteil der langen Inkubationszeiten. Eine Inkubation von mindestens zwei Stunden ist hier erforderlich.

Aufgabe der Erfindung ist es daher, ein Verfahren zu schaffen, welches nicht kompetitiv abläuft, ohne radioaktives Material auskommt, nur kurze Zeiten benötigt und bei einfachster Testführung hohe Genauigkeit aufweist.

Gelöst wird diese Aufgabe erfindungsgemäß durch ein Verfahren zur Bestimmung der Bindungskapazität des Thyroxin bindenden Globulins (TBG) nach dem Prinzip des heterogenen Enzymimmunassays, welches dadurch gekennzeichnet ist, daß man die zu untersuchende Serumprobe mit enzymmarkiertem $T_4$ oder $T_3$ und immobilisiertem Antikörper gegen TBG oder $T_4$ inkubiert, die Phasen trennt und das Markierungsenzym in einer der Phasen mißt.

Das erfindungsgemäße Verfahren läßt sich bei einfachster Testführung in kurzer Zeit mit maximal 15 Minuten Inkubationszeit durchführen.

Im Gegensatz zu allen bekannten heterogenen Verfahren zur Bestimmung der Bindungskapazität von TBG, die mit immobilisierten Anti-$T_4$-Antikörpern arbeiten, so daß bei ihrer Durchführung TBG bzw. Anti-$T_4$-Antikörper um das $T_4$ bzw. ein $T_4$-Konjugat konkurrieren, arbeitet die bevorzugte Ausführungsform des erfindungsgemäßen Verfahren mit immobilisierten Anti-TBG-Antikörpern. Es ist auch als sehr überraschend anzusehen, daß bei der erfindungsgemäßen Verfahrensführung exakte Resultate erhalten werden, da es bekannt war, daß enzymmarkiertes $T_3$ oder $T_4$ nicht von den Serumproteinen (TBG) gebunden wird. Auf diesen speziellen Eigenschaften beruht sowohl das der FR—A—81 17 997 (Corning) als auch das in der DE—A—28 25 650 beschriebene Verfahren.

Als Enzymmarkierung für das $T_3$ oder $T_4$ können im Rahmen der Erfindung alle hierfür geeigneten, leicht bestimmbaren Enzyme verwendet werden, wie sie dem Fachmann zahlreich bekannt sind. Bevorzugte Beispiele hierfür sind Peroxidase und β-Galactosidase. Vorzugsweise wird das Enzym über ein Distanzstück (Spacer) an das Hormon gekoppelt, wobei die Distanzlänge 3 bis 8 C-Atome, vorzugsweise 4 bis 5 C-Atome betragen soll. Die Kopplung erfolgt beispielsweise über einen aktiven Ester des Hormons oder des Hormonderivats. Als Spacer für die Verbindung von $T_4$ bzw. $T_3$ und Markierungsenzym werden besonders bevorzugt Aminobuttersäure oder Glycylglycin und zwar zweckmäßig in Form ihrer Hydroxysuccinimidester verwendet.

Das enzymmarkierte $T_4$ oder $T_3$, im folgenden als Konjugat bezeichnet, wird beim erfindungsgemäßen Verfahren in solcher Konzentration eingesetzt, daß $T_4$ nicht merklich aus dem im Serum vorhandenen TBG-$T_4$-Komplex verdrängt wird. Bevorzugt wird deshalb das Konjugat im Unterschuß gegenüber der zu erwartenden TBG-Gesamtkonzentration eingesetzt.

Als Konjugat wird vorzugsweise ein solches verwendet, bei dem das molekulare Verhältnis von $T_4$ bzw. $T_3$ zum Markierungsenzym, wie insbesondere β-Galactosidase 1 bis 5:1 beträgt.

Aufgrund seiner größeren Bindungskapazität wird beim Verfahren der Erfindung bevorzugt mit einem $T_4$-haltigen Konjugat gearbeitet, was zu einer besseren Empfindlichkeit führt. Die Erfindung läßt sich jedoch in gleicher Weise auch mit $T_3$-Konjugat durchführen.

Als Anti-TBG-Antikörper kann ein polyklonaler wie auch ein monoklonaler Antikörper, wobei letzterer bevorzugt wird, oder ein Fragment eines solchen Antikörpers, verwendet werden. Die Immobilisierung des Antikörpers kann in bekannter Weise erfolgen derart, daß der Antikörper direkt an den Träger gebunden wird oder unter Ausnützung seiner immunologischen Bindefähigkeit über einen immobilisierten Anti-Antikörper oder ein Fragment desselben fixiert wird. Die Immobilisierung des Anti-TBG-Antikörpers über einen an die feste Phase gebundenen Anti-Antikörper oder ein Fragment davon hat den großen Vorteil, daß der wertvolle Anti-TBG-Antikörper praktisch zu 100% aktiv gebunden wird und damit in geringerer Menge

2

eingesetzt werden kann als bei direkter Fixierung, die immer mit einem gewissen Aktivitätsverlust verbunden ist.

Besonders bevorzugt ist hierbei eine Ausführungsform, bei welcher ein gegen den Fc-Teil des Anti-TBG-Antikörpers gerichteter immobilisierter Antikörper verwendet wird, der mit dem gelösten Anti-TBG-Antikörper unter Fixierung des letzteren umgesetzt wird. Die obigen Ausführungen gelten analog, wenn anstelle von Anti-TBG-Antikörper Anti-$T_4$-Antikörper verwendet wird.

Das Verfahren der Erfindung kann zweistufig durchgeführt werden, wobei in erster Stufe die Probe mit dem Konjugat inkubiert und in zweiter Stufe dann mit dem immobilisierten Antikörper gegen TBG oder $T_4$ inkubiert wird. Soweit hierbei Anti-$T_4$-Antikörper verwendet werden, wird die zweite Inkubation zweckmäßig nicht länger als 5 Minuten, vorzugsweise 0,5 bis 2 Minuten, durchgeführt. Bei darüberhinausgehender Dauer der zweiten Inkubation können Konkurrenzreaktionen auftreten, welche die Genauigkeit herabsetzen.

Bei Verwendung von Antikörpern gegen TBG wird das Verfahren bevorzugt auch einstufig durchgeführt, derart, daß alle Komponenten, auch die feste Phase gleichzeitig zusammengegeben werden.

Die folgenden Beispiele erläutern die Erfindung weiter in Verbindung mit der beigefügten Zeichnung. In dieser stellen dar:

Fig. 1 eine Eichkurve, die mit dem erfindungsgemäßen Verfahren mit Proben, deren TBG-Bindungskapazität bekannt war, erhalten wurde und

Fig. 2 einen Vergleich der erfindungsgemäß erhaltenen Werte (Ordinate) mit den Werten eines bekannten Verfahrens (Abszisse).

Beispiel 1
1. Reagenzien:

a) Puffer:
    100 mmol/l Hepes
    2 mmol/l Mg-Aspartat
    1% RSA
    0,9% NaCl
    0,2% oberflächenaktives Mittel (Tween®), pH=7,25 (37°C)

b) $T_4$-β-Galactosidase:
    600 mU/ml Puffer

c) monoklonaler Antikörper (gerichtet gegen TBG); 108 µg/ml
    (Die Entwicklung des monoklonalen Antikörpers erfolgte nach der Methode von Köhler und Milstein (Eur. J. Immunol. 6, 292- (1976), als Immunogen wurde TBG verwendet).

d) immobilisierter Antikörper (gerichtet gegen Fc-Teil des monoklonalen Antikörpers).

Der Antikörper wurde durch Immunisierung von Schafen mit Fc-Teilen von Maus-Immunglobulinen nach üblichen Verfahren gewonnen. Das Antiserum wurde über Ammoniumsulfatfällung und Chromatographie an DEAE-Cellulose aufgereinigt.

Die Fixierung des Antikörpers auf Filterpapier erfolgte nach der von P. Gemeiner und M. Pasteka in Applied Biochemistry and Biotechnology 8, 381—393 (1983) beschriebenen Methode zur Herstellung von Protein-Cellulose-Konjugaten durch reduktive Alkylierung von perjodatoxidierter Cellulose. Angeboten wurden 15 mg gereinigter Antikörper/g Papier.

Nach Kupplung wurde das Papier mit $H_2O$ und RSA-haltigem Phosphatpuffer gewaschen und bei 35°C getrocknet.

e) Chlorphenolrotgalactosid (CPRG)-Vlies:
    0,25 µmol/5 mg Papier, hergestellt nach DE—A—33 45 748.
    Testdurchführung:
      Serum wird im Volumenverhältnis von 1:75 mit folgendem Gemisch verdünnt:
    172 µl Puffer
    30 µl $T_4$-β-Galactosidaselösung
    20 µl MAK<TBG>-Lösung (monoklonaler Antikörper gegen TBG)

Nach 5 Minuten Inkubation bei 37°C läßt man 50 µl des Gemisches von 10 mg des gemäß d) hergestellten Kupplungs-Papieres aufsaugen und inkubiert weitere 5 Minuten bei 37°C. Durch anschließende Zentrifugation wird die Flüssigkeit, die den ungebundenen Anteil an $T_4$-β-Galactosidase enthält, vom Papier abgetrennt, über das Chlorphenolrotgalactosid-Vlies geleitet und die Enzymkinetik (mE/min) bei 37°C und 578 nm in einer Mikroküvette mit einem Füllvolumen von 27 µl und einer Schichtdicke von 0,6 cm gemessen.

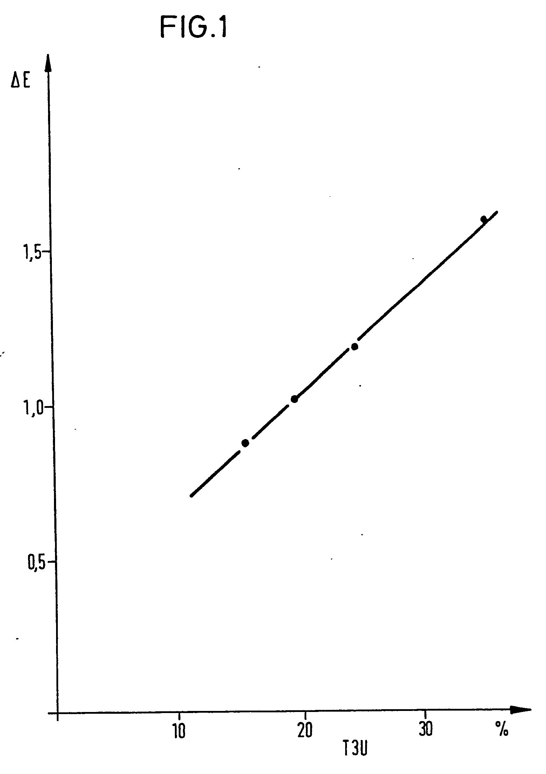

Die Umrechnung des Meßsignals in TBG-Bindungskapazitätswerte erfolgt über eine Eichkurve, die mit Proben, deren TBG-Wert bekannt ist, erhalten wurde (Fig. 1).

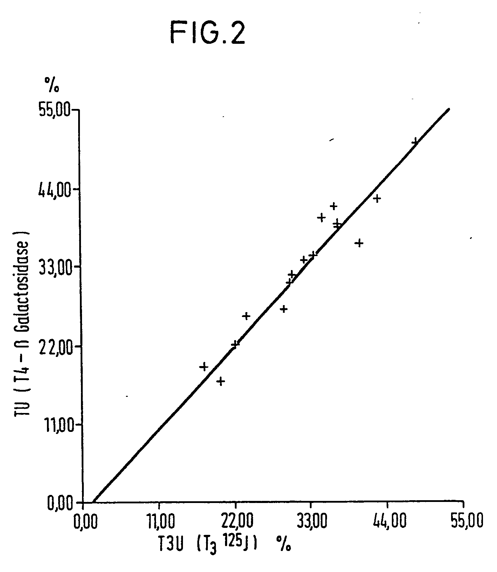

Fig. 2 zeigt einen Methodenvergleich:

Abszisse: Thyroxin-Bindungskapazität im Serum (%), erhalten mit einem Radioassay

Ordinate: TBG-Bindungskapazität (%), erhalten gemäß Beispiel.

Stand. Hauptkomp. Analyse:

Y=1.325+1.049×Korrelationskoeffizient: 0.964.

2. Herstellung des $T_4$-β-Galactosidase-Konjugats mit Aminobuttersäure als Spacer

N-tertiär-Butyloxycarbonyl-Thyroxin (BOC-$T_4$) wird aus Thyroxin-Na-Salz und Di-tertiärbutyldicarbonat analog der in Eu-A—01 08 400 angegebenen Vorschrift hergestellt.

N-tertiär-Butyloxycarbonyl-thyroxinyl-hydroxysuccinimidester (BOC-$T_4$-OSu) wird aus 0,5 mmol BOC-$T_4$, 3,6 mmol N-Hydroxysuccinimid und 3,6 mmol Dicyclohexyldiimid analog der Vorschrift zur Darstellung von BOC-Phenylalanin-OSu hergestellt (Houben-Weyl, Methoden der anorganischen Chemie, Band XV/2, Jahrgang 74, S. 150).

Der nach Eindampfen erhaltene Rückstand wird in Essigester gelöst und durch Zugabe von Petrolether ausgefällt.

N-tertiär-Butyloxycarbonyl-thyroxinyl-aminobuttersäure-hydroxy-succinimidester (BOC-$T_4$-Abs-OSu) wird folgendermaßen dargestellt:

Zu einer Lösung von 0,5 g BOC-$T_4$-OSu in 8 ml Dioxan wird eine Lösung von 0,27 g Aminobuttersäure in 0,5 ml 0,1 m Phosphatpuffer pH 8,5 getropft. Nach zweistündiger Reaktionszeit wird angesäuert und mit Essigester ausgeschüttelt. Die Essigesterphase wird mit $H_2O$ gewaschen, getrocknet und eingedampft.

Der Rückstand wird in 20 ml Ethylenglycoldimethylether gelöst und mit 0,07 g N-Hydroxysuccinimid und 0,12 g Dicyclohexylcarbodiimid versetzt. Nach 12stündiger Reaktionszeit wird abfiltriert. Das Lösungsmittel wird abgedampft, der Rückstand in Essigester gelöst und BOC $T_4$ Abs OSu mit Petrolether ausgefällt.

DC (HPTLC, RP 18; Laufmittel: Nitromethan/Ethanol 9:1) $R_f$: 0,77 $^1$H-NMR ($d_6$-Dimethylsulfoxid), δ (ppm): 1,32 (s, 9H), 1,77 (m, 4H), 2,80 (s, 4H), 7,03 (s, 2H), 7,79 (s, 2H).

15,8 mg β-Galactosidase werden in 2,5 ml 0,01 M Phosphatpuffer, pH=7,0 gelöst. Unter Eiskühlung werden 0,307 ml einer 0,1%igen Lösung von BOC-$T_4$-Abs-OSu langsam zugegeben. Das erhaltene Konjugat wird über Gelfiltration entsalzt und über Spherosil gekuppeltes TBG affinitätschromatographisch gereinigt.

Beispiel 2

a) Puffer: 110 mmol/l Hepes, 2,2 mmol/l Mg-Aspartat, 1% RSA, 0,9% NaCl, 0,2% Tween®, pH=7,25 (37°C)

b) $T_3$-β-Galactosidase: 125 mU/ml Puffer

c) Auf Papier immobilisierter Antikörper, gerichtet gegen TBG

Die Antikörper wurden durch Immunisierung von Schafen mit TBG nach üblichen Verfahren gewonnen. Die Antikörper wurden nach Ammoniumsulfatfällung und DEAE-Cellulose-Chromatographie zusätzlich über an Spherosil gekuppeltes TBG immunsorptiv gereinigt. Die Fixierung des Antikörpers an Papier erfolgte wie im Beispiel 1 beschreiben. Angeboten wurden 20 mg gereinigter Antikörper/g Papier.

d) Chlorphenolrotgalactosidlösung (5 mmol/l)

Puffer: 100 mmol/l Hepes, 2 mmol/l Mg-Aspartat, 0,5% RSA, 0,9% NaCl, 0,2% Tween®.

Testdurchführung:

Serum wird im Volumenverhältnis von 1:10 mit $T_3$-β-Galactosidaselösung verdünnt. 50 µl dieses Gemisches werden von 10 mg des TBG-Antikörper tragenden Papiers aufgesaugt und 10 Minuten bei 37°C inkubiert. Zur Entfernung von ungebundener $T_3$-β-Galactosidase wird das Papier dreimal mit je 100 µl Puffer gewaschen, wobei nach jedem Waschvorgang die Waschlösung durch Zentrifugation entfernt wird. Anschließend wird das Papier mit 50 µl Chlorphenolrotgalactosidlösung getränkt. Nach 5 Minuten Inkubation bei 37°C wird die gebildete Farbstofflösung durch Zentrifugation vom Papier abgetrennt und in einer Mikroküvette mit einem Füllvolumen von 27 µl und einer Schichtdicke von 0,6 cm bei einer Wellenläng von 578 nm gemessen.

Die gemessene Extinktion ist proportional der Bindungskapazität der Probe.

| Probe | $\Delta E_{578}$nm (bezogen auf eine Schichtdicke von 1 cm) |
|---|---|
| Serum mit niedriger Bindungskapazität<br>T3U (Radioassay): 59%<br>TBG <2 µg/ml | 357 mE |
| Serum mit normaler Bindungskapazität<br>T3U: 28%<br>TBG: 16 µg/ml | 1351 mE |

Herstellung des $T_3$-β-Galactosidasekonjugats Die Herstellung des N - tertiär - Butyloxycarbonyl - trijodthyronins (BOC-$T_3$) und des N - tertiär - Butyloxycarbonyltrijodthyronin - hydroxysuccinimidesters (BOC-$T_3$-OSu) erfolgt analog wie in Beispiel 1 beschrieben.

BOC-$T_3$-OSu: DC (HPTLC, RP 18, Laufmittel: Nitromethan/Ethanol 9:1) $R_f=0,6$

Die Umsetzung der β-Galactosidase mit BOC-$T_3$-OSu und die anschließende Aufreinigung wird ebenfalls wie im Beispiel 1 durchgeführt.

Beispiel 3
Reagenzien:
a) Puffer:

100 mmol/l Hepes
2 mmol/l Mg-Aspartat
1% RSA (Rinderserumalbumin)
0,9% NaCl
0,2% Tween®, pH=7,25 (37°C)

b) $T_4$-β-Galactosidase: 170 mU/ml Puffer (Herstellung nach Beispiel 1)

c) Auf Papier immobilisierter Anti-$T_4$-Antikörper (S<$T_4$>-Papier).

Die Antikörper wurden durch Immunisierung von Schafen mit $T_4$, das über Glutardialdehyd an Rinderserumalbumin gebunden ist, nach üblichem Verfahren gewonnen.

Das Antiserum wurde über Ammoniumsulfatfällung und Chromatographie an DEAE-Cellulose aufgereinigt.

Die Fixierung des Antikörpers an Papier erfolgte analog, wie im Beispiel 1 beschrieben.

Eingesetzt wurden 10 mg gereinigter Antikörper/g Papier.

d) Chlorphenolrotgalactosid (CPRG)-Vlies: 0,25 µmol/5 mg Papier (wie im Beispiel 1)
Testdurchführung:
Serum wird im Volumenverhältnis von 1:20 mit folgendem Gemisch verdünnt:

140 µl Puffer a)
50 µl $T_4$-ß-Galactosidaselösung b)

Nach 5 Minuten Inkubation bei 37°C läßt man 50 µl des Gemisches von 10 mg des S<$T_4$>-Papiers c) aufsaugen und inkubiert weitere 5 Minuten bei 37°C. Durch anschließende Zentrifugation wird die Flüssigkeit, die die Komplexe aus TBG und $T_4$-β-Galactosidase enthält, vom Papier abgetrennt, über das Chlorphenolrotgalactosid-Vlies d) geleitet und die Enzymkinetik (mE/min) bei 37°C und 578 nm in einer Mikroküvette mit einem Füllvolumen von 27 µl und einer Schichtdicke von 0,6 cm gemessen.

Die Umrechnung des Meßsignals in T-uptake-Werte erfolgt über eine Eichkurve, die mit Proben, deren uptake-Wert bekannt ist, erhalten wird

| Serum | $\Delta E_{578}$ nm bezogen auf Schichtdicke von 1 cm | T-uptake % | $T_3$U (Radioassay) % | TBG (Elisa) µg/ml |
|---|---|---|---|---|
| 1 | 665 mE | 42,3 | 40,1 | 5,6 |
| 2 | 1028 mE | 28 | 28,2 | 14,6 |
| 3 | 947 mE | 30,5 | 30,0 | 12,0 |
| 4 | 1129 mE | 25 | 18,3 | 18,6 |

# EP 0 190 765 B1

**Patentansprüche**

1. Verfahren zur Bestimmung der Bindungskapazität des Thyroxin bindenden Globulins (TBG) nach dem Prinzip des heterogenen Enzymimmunoassays, dadurch gekennzeichnet, daß man die zu untersuchende Serumprobe mit einem $T_4$- oder $T_3$-Enzymkonjugat, bei dem $T_4$ bzw. $T_3$ und Enzym über einen Spacer gebunden sind und immobilisiertem Antikörper gegen TBG oder $T_4$ bzw. gelöstem Antikörper gegen TBG oder $T_4$ und immobilisiertem Antikörper gegen den Fc-Teil des Antikörpers gegen TBG oder $T_4$ inkubiert, die Phasen trennt und das Markierungsenzym in einer der Phasen mißt.

2. Verfahren nach Anspruch 1, dadurch gekennzeichnet, daß man monoklonalen Anti-TBG-Antikörper verwendet.

3. Verfahren nach einem der Ansprüche 1 oder 2, dadurch gekennzeichnet, daß ein Spacer mit einer Länge von 3 bis 8 C-Atomen verwendet wird.

4. Verfahren nach Anspruch 3, dadurch gekennzeichnet, daß als Spacer Aminobuttersäure oder Glycylglycin verwendet wird.

5. Verfahren nach einem der vorhergehenden Ansprüche, dadurch gekennzeichnet, daß man das Verfahren zweistufig durchführt und in erster Stufe die Probe mit dem enzymmarkierten $T_3$ oder $T_4$ inkubiert und in zweiter Stufe mit dem immobilisierten Antikörper gegen $T_4$ oder TBG inkubiert.

6. Verfahren nach Anspruch 5, dadurch gekennzeichnet, daß man bei Verwendung von Anti-$T_4$-Antikörper die zweite Inkubationsstufe nicht länger als 5 Minuten durchführt.

**Revendications**

1. Procédé de détermination de la capacité de fixation de la globuline fixant la thyroxine (TBG) par le principe du dosage enzymo-immunologique hétérogène, caractérisé en ce qu'on fait incuber l'échantillon de sérum à étudier avec un conjugué $T_4$- ou $T_3$-enzyme, dans lequel la $T_4$ ou la $T_3$ et l'enzyme sont liés par l'intermédiaire d'un spacer, et avec un anticorps immobilisé contre la TBG ou la $T_4$ ou un anticorps contre la TBG ou la $T_4$ dissous et un anticorps immobilisé contre la partie Fc de l'anticorps contre la TBG ou la $T_4$, en ce qu'on sépare les phases et en ce qu'on mesure l'enzyme de marquage dans une des phases.

2. Procédé selon la revendication 1, caractérisé en ce qu'on utilise un anticorps anti-TBG monoclonal.

3. Procédé selon l'une des revendications 1 ou 2, caractérisé en ce qu'on utilise un spacer d'une longueur de 3 à 8 atomes de carbone.

4. Procédé selon la revendication 1, caractérisé en ce qu'on utilise comme spacer l'acide aminobutyrique ou la glycylglycine.

5. Procédé selon l'une des revendications précédentes, caractérisé en ce qu'on effectue le procédé en deux étapes, et en ce que, dans la première étape, on fait incuber l'échantillon avec la $T_3$ ou la $T_4$ marquée par l'enzyme et, dans la deuxième étape, on le fait incuber avec l'anticorps immobilisé contre la $T_4$ ou la TBG.

6. Procédé selon la revendication 5, caractérisé en ce que, dans le cas de l'utilisation d'anticorps anti-$T_4$, on effectue la deuxième étape d'incubation pendant une durée non supérieure à 5 minutes.

**Claims**

1. Process for the determination of the binding capacity of thyroxine-binding globulin (TBG) according to the principle of heterogeneous enzyme immune assay, characterised in that one incubates the serum sample to be investigated with an enzyme-labelled $T_4$ or $T_3$, in which $T_4$ or $T_3$ and enzyme are bound via a spacer, and immobilised antibody against TBG or $T_4$ or dissolved antibody against TBG or $T_4$ and immobilised antibody against the Fc part of the antibody against TBG or $T_4$, separates the phases and measures the labelling enzyme in one of the phases.

2. Process according to claim 1, characterised in that one uses monoclonal anti-TBG antibodies.

3. Process according to one of claims 1 or 2, characterised in that a spacer with a length of 3 to 8 C-atoms is used.

4. Process according to claim 3, characterised in that aminobutyric acid or glycylglycine is used as spacer.

5. Process according to one of the preceding claims, characterised in that one carries out the process in two stages and, in the first stage, incubates the sample with the enzyme-labelled $T_3$ or $T_4$ and, in the second stage, incubates with the immobilised antibody against $T_4$ or TBG.

6. Process according to claim 5, characterised in that, in the case of the use of anti-$T_4$ antibody, one carries out the second incubation stage for not longer than 5 minutes.

## FIG.1

# FIG.2